# EUROPEAN PATENT SPECIFICATION

(11) **EP 2 898 831 B1**
(45) Date of publication and mention of the grant of the patent: **21.08.2019**
(21) Application number: 14200600.6
(22) Date of filing: 30.12.2014
(51) Int. Cl.: A61B 8/00, A61B 8/08, G01S 15/89, G01S 7/52, G06T 15/00, A61B 8/06

(54) **METHOD AND ULTRASOUND APPARATUS FOR DISPLAYING ULTRASOUND IMAGE**
VERFAHREN UND ULTRASCHALLVORRICHTUNG ZUR ANZEIGE VON ULTRASCHALLBILDERN
PROCÉDÉ ET APPAREIL À ULTRASONS POUR AFFICHER UNE IMAGE ULTRASONORE

(30) Priority: 28.01.2014 KR 20140010883
(43) Date of publication of application: 29.07.2015
(73) Proprietor: Samsung Medison Co., Ltd., Gangwon-do 250-870 (KR)
(72) Inventor: OH, Dong-hoon, Gangwon-do (KR); HYUN, Dong-gyu, Gangwon-do (KR)
(74) Representative: Frey, Sven Holger

(56) References cited:
- EP-A1- 2 705 798
- EP-A2- 2 030 570
- EP-A2- 2 333 576
- US-A1- 2011 063 288
- US-A1- 2012 116 219
- US-A1- 2012 184 849
- R. OYAMA ET AL: "Towards improved ultrasound-based analysis and 3D visualization of the fetal brain using the 3D Slicer", ULTRASOUND IN OBSTETRICS AND GYNECOLOGY, vol. 42, no. 5, 2 November 2013 (2013-11-02), pages 609-610, XP055196768, ISSN: 0960-7692, DOI: 10.1002/uog.12484

## Description

### BACKGROUND

### 1. Field

The present disclosure relates to ultrasound image display methods and ultrasound apparatuses for displaying an ultrasound image corresponding to a partial range of image pixel information acquired from ultrasound image data as a three-dimensional (3D) image having height values.

### 2. Description of the Related Art

An ultrasound diagnosis apparatus transfers an ultrasound signal from a surface of an object to a predetermined region in a body of the object, and obtains a tomogram of a soft tissue or an image of a bloodstream by using information of an ultrasound signal reflected from a tissue in the body.

The ultrasound diagnosis apparatus is small and inexpensive and may display images in real time. Also, since the ultrasound diagnosis apparatus is very safe due to there being no exposure to X-rays or the like, the ultrasound diagnosis apparatus is widely used along with other image diagnosis apparatuses such as an X-ray diagnosis apparatus, a computerized tomography (CT) scanner, a magnetic resonance imaging (MRI) apparatus, and a nuclear medicine diagnosis apparatus.

Since values measured by the ultrasound diagnosis apparatus are closely related to a lesion diagnosis or the like, accuracy of the values is required. Therefore, there is a need for a system that enables a user to accurately understand an ultrasound image.

EP 2 333 576 A2 refers to an ultrasound system and a method for providing a 3D ultrasound image. The ultrasound system comprises an ultrasound data acquisition unit configured to transmit and receive ultrasound signals to and from a target object to output a plurality of ultrasound data, a user input unit configured to receive first input information for defining a region of interest (ROI) from a user, and a processing unit in communication with the ultrasound data acquisition unit and the user input unit. The processing unit is configured to form volume data based on the plurality of ultrasound data, define the ROI in the volume data in response to the first input information, render volume data corresponding to the ROI to form a 3D ultrasound image, define the sub ROI in the volume data based on the second input information, and render volume data corresponding to the sub ROI to form a sub 3D ultrasound image.

US 2012/184849 A1 discloses an ultrasound diagnosis apparatus providing a map of an interest index. The ultrasound diagnosis apparatus includes a calculating unit for calculating a mechanical index (MI) corresponding to a depth value in a direction in which ultrasound travels from an ultrasound output part of a transmission transducer and a visualization unit for generating an MI map in which a relationship between the calculated MI and the depth value is visualized in the form of a graph.

XP0055196768 "Towards improved ultrasound-based analysis and 3D visualization of the fetal brain using the 3D Slicer" by R. Oyama et al. provides a workflow for semi-automated segmentation and 3D visualization of fetal ultrasound volumes in the second trimester using the 3D Slice open source software. The workflow allows quantitative image analysis of the choroid plexus and cerebrum from 3D ultrasound images.

EP 2 705 798 A1, which was published after the priority date of the present application, describes a method of displaying stereoscopic information related to an ultrasound sectional plane of a target object. The method includes setting a line of interest on the target object, obtaining brightness information of the ultrasound sectional plane of the target object along the set line of interest, converting the obtained brightness information into height information, and displaying the stereoscopic information related to the ultrasound sectional plane of the target object based on the height information.

### SUMMARY

The present invention is defined by the appended claims.

Aspects, embodiments, examples, methods and apparatuses of the present disclosure which do not fall within the scope of the appended claims do not form part of the present invention. One or more exemplary embodiments include ultrasound image display methods and ultrasound apparatuses for extracting ultrasound image data corresponding to a partial range of image pixel information selected by a user and displaying a partial ultrasound image as a three-dimensional (3D) image having a height value by using the extracted ultrasound image data.

Additional aspects will be set forth in part in the description which follows and, in part, will be apparent from the description, or may be learned by practice of the presented exemplary embodiments.

According to one or more exemplary embodiments, an ultrasound image display method includes: acquiring ultrasound image data of an object; receiving a selection of at least one range of image pixel information acquired from the ultrasound image data; extracting partial image ultrasound image data corresponding to the at least one range from among the ultrasound image data; and three-dimensionally displaying an ultrasound image corresponding to the at least one range by using the partial ultrasound image data.

The ultrasound image data may include at least one of brightness (B) mode image data, spectral Doppler image data, color Doppler image data, elasticity image data, and motion (M) mode image data.

The image pixel information acquired from the ultrasound image data may include at least one of brightness information, speed information, color information, elasticity information, amplitude information of a sound reflection signal, and sound impedance information.

The receiving of the selection of the at least one range may include: displaying an image pixel information map, in which the image pixel information is gradually represented, on a screen; and receiving a user input for selecting the at least one range through the image pixel information map.

The image pixel information map may include at least one of a gray scale map, a color scale map, an elasticity map, and a speed map.

The receiving of the selection of the at least one range may include: displaying a setting window for setting the at least one range on a screen; and receiving the selection of the at least one range through the setting window.

The three-dimensional displaying of the ultrasound image corresponding to the at least one range may include converting image pixel information acquired from the partial ultrasound image data into height values.

The converting of the image pixel information into the height values may include determining a sign of the height values based on movement direction information of a tissue or a bloodstream when the partial ultrasound image data is color Doppler image data or spectral Doppler image data.

The three-dimensional displaying of the ultrasound image corresponding to the at least one range may include two-dimensionally displaying other images except the ultrasound image corresponding to the at least one range among the entire ultrasound image displayed based on the ultrasound image data.

The three-dimensional displaying of the ultrasound image corresponding to the at least one range may include transparently or semi-transparently displaying other images except the ultrasound image corresponding to the at least one range among the entire ultrasound image displayed based on the ultrasound image data.

The three-dimensional displaying of the ultrasound image corresponding to the at least one range may include three-dimensionally displaying the ultrasound image corresponding to the at least one range by using a light source-based rendering method.

The three-dimensional displaying of the ultrasound image corresponding to the at least one range may include: receiving a selection of at least one rendering method among a plurality of rendering methods; and three-dimensionally displaying the ultrasound image corresponding to the at least one range by using the selected rendering method.

The receiving of the selection of the at least one range may include receiving a selection of a first range and a second range of the image pixel information acquired from the ultrasound image data.

According to one or more exemplary embodiments, an ultrasound apparatus includes: an ultrasound image data acquiring unit configured to acquire ultrasound image data of an object; a user input unit configured to receive a selection of at least one range of image pixel information acquired from the ultrasound image data; and a display configured to extract partial image ultrasound image data corresponding to the at least one range from among the ultrasound image data, and three-dimensionally displaying an ultrasound image corresponding to the at least one range by using the partial ultrasound image data.

The controller may control the display to display an image pixel information map in which the image pixel information is gradually represented, and the user input unit may receive a user input for selecting the at least one range through the image pixel information map.

The controller may control the display to display a setting window for setting the at least one range, and the user input unit may receive the selection of the at least one range through the setting window.

The controller may convert image pixel information acquired from the partial ultrasound image data into height values.

The controller may determine a sign of the height values based on movement direction information of a tissue or a bloodstream when the partial ultrasound image data is color Doppler image data or spectral Doppler image data.

The controller may control the display to two-dimensionally display other images except the ultrasound image corresponding to the at least one range among the entire ultrasound image displayed based on the ultrasound image data.

The controller may control the display to transparently or semi-transparently display other images except the ultrasound image corresponding to the at least one range among the entire ultrasound image displayed based on the ultrasound image data.

The controller may control the display to three-dimensionally display the ultrasound image corresponding to the at least one range by using a light source-based rendering method.

The user input unit may receive a selection of at least one rendering method among a plurality of rendering methods, and the controller may control the display to three-dimensionally display the ultrasound image corresponding to the at least one range by using the selected rendering method.

The user input unit may receive a selection of a first range and a second range of the image pixel information acquired from the ultrasound image data.

### BRIEF DESCRIPTION OF THE DRAWINGS

These and/or other aspects will become apparent and more readily appreciated from the following description of the exemplary embodiments, taken in conjunction with the accompanying drawings in which:
FIG. 1 is a diagram illustrating an ultrasound apparatus and a general ultrasound image acquired by the ultrasound apparatus, according to an exemplary embodiment;
FIG. 2 is a flowchart of an ultrasound image display method according to an exemplary embodiment;
FIGS. 3A and 3B are diagrams illustrating a three-dimensional (3D) ultrasound image having height values, according to an exemplary embodiment;
FIG. 4 is a diagram illustrating an operation of receiving a selection of at least one range through an image pixel information map, performed by the ultrasound apparatus, according to an exemplary embodiment;
FIG. 5 is a diagram illustrating a setting window for setting a partial range of image pixel information, according to an exemplary embodiment;
FIGS. 6A and 6B are diagrams illustrating a 3D brightness (B) mode image obtained by converting ultrasound image data corresponding to a predetermined brightness range, according to an exemplary embodiment;
FIGS. 7A and 7B are diagrams illustrating a 3D color Doppler image obtained by converting ultrasound image data corresponding to a predetermined color range, according to an exemplary embodiment;
FIG. 8 is a diagram illustrating an operation of receiving a selection of a plurality of ranges of image pixel information, performed by the ultrasound apparatus, according to an exemplary embodiment;
FIGS. 9A, 9B, 9C, 9D, and 9E are diagrams illustrating an operation of processing other images, except an ultrasound image corresponding to a selected range among the entire ultrasound image, to be transparent, performed by the ultrasound apparatus, according to an exemplary embodiment;
FIGS. 10A, 10B, 10C, 10D, and 10E are diagrams illustrating an operation of selecting a rendering method, performed by the ultrasound apparatus, according to an exemplary embodiment; and
FIGS. 11 and 12 are block diagrams of the ultrasound apparatus according to an exemplary embodiment.

### DETAILED DESCRIPTION

Throughout the specification, an "ultrasound image" refers to an image of an object obtained by using an ultrasound signal. The object may refer to a part of a body. For example, the object may include organs such as liver, heart, nuchal translucency (NT), brain, breast, and abdomen, or embryo.

Throughout the specification, a "user" may be, but is not limited to, a medical expert including a doctor, a nurse, a medical laboratory technologist, or a sonographer.

Reference will now be made in detail to exemplary embodiments, examples of which are illustrated in the accompanying drawings. The inventive concept may, however, be embodied in many different forms and should not be construed as being limited to the exemplary embodiments set forth herein. In addition, portions irrelevant to the description of the exemplary embodiments will be omitted in the drawings for a clear description of the exemplary embodiments, and like reference numerals will denote like elements throughout the specification.

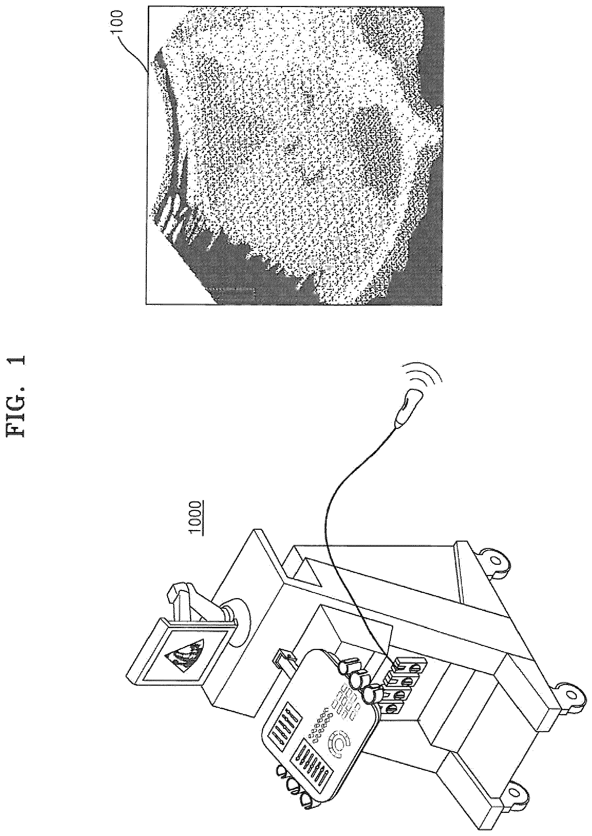

FIG. 1 is a diagram illustrating an ultrasound apparatus 1000 and a general ultrasound image acquired by the ultrasound apparatus 1000, according to an exemplary embodiment.

As illustrated in FIG. 1, the ultrasound apparatus 1000 may transmit an ultrasound signal to an object. Then, the ultrasound apparatus 1000 may receive an ultrasound echo signal reflected from the object and generate an ultrasound image.

In this specification, the ultrasound image may be implemented in various ways. For example, the ultrasound image may include, but is not limited to, at least one of a brightness (B) mode image representing, by brightness, a magnitude of an ultrasound echo signal reflected from the object, a color Doppler image representing, by color, a speed of a moving object by using the Doppler effect, a spectral Doppler image representing an image of a moving object in the form of a spectrum by using the Doppler effect, a motion (M) mode image representing a time-dependent motion of an object at a predetermined position, and an elasticity mode image representing a difference between a reaction of an object when compression is applied to the object and a reaction of the object when compression is not applied to the object, by using an image. Also, according to an exemplary embodiment, the ultrasound image may be a two-dimensional (2D) image, a three-dimensional (3D) image, or a four-dimensional (4D) image.

For example, the ultrasound apparatus 1000 may convert ultrasound image data into brightness values and display an ultrasound image of the object as a 2D B mode image 100. In some cases, the user may have difficulty in clearly detecting a brightness difference in the B mode image 100.

Also, even when the ultrasound apparatus 1000 converts the ultrasound image data into color values and displays a 2D color mode image, the user may have difficulty in accurately detecting colors due to an individual difference such as a color sensitivity difference, an optical illusion, color weakness, or color blindness.

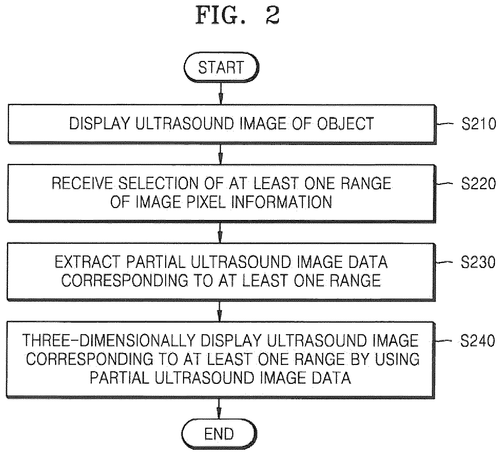

Hereinafter, a method of displaying a portion of an ultrasound image as a 3D image having height values to enable the user to reduce an analysis error in the ultrasound image will be described in detail with reference to FIG. 2,

FIG. 2 is a flowchart of an ultrasound image display method according to an exemplary embodiment.

Referring to FIG. 2, in operation S210, the ultrasound apparatus 1000 may acquire ultrasound image data of an object.

According to an exemplary embodiment, the ultrasound apparatus 1000 may directly generate ultrasound image data of the object or may receive ultrasound image data of the object from outside thereof. For example, the ultrasound apparatus 1000 may transmit an ultrasound signal to the object, receive an ultrasound response signal reflected from the object, and generate ultrasound image data. Also, the ultrasound apparatus 1000 may receive ultrasound image data from an external server or an external device.

According to an exemplary embodiment, the ultrasound image data may include, but is not limited to, at least one of B mode image data, spectral Doppler image data, color Doppler image data, elasticity image data, and M mode image data. The color Doppler image data may include at least one of Doppler image data of a bloodstream and Doppler image data of a tissue.

In operation S220, the ultrasound apparatus 1000 may receive a selection of at least one range of image pixel information acquired from the ultrasound image data. For example, the ultrasound apparatus 1000 may receive a selection of a first range of the image pixel information acquired from the ultrasound image data.

According to an exemplary embodiment, the image pixel information acquired from the ultrasound image data, which represents values of pixels displayed on a screen, may include, but is not limited to, at least one of brightness information (e.g., 0 to 255), speed information, color information (e.g., -127 to 127), and elasticity information (e.g., modulus of elasticity and strain). According to an exemplary embodiment, the speed information may include, but is not limited to, speed of movement of a tissue or a bloodstream, speed variation, and speed variance. Also, the image pixel information acquired from the ultrasound image data may include at least one of amplitude information of a sound reflection signal and sound impedance information.

For example, the ultrasound apparatus 1000 may receive a selection of a first range (e.g., 100 to 150) among all brightness values (e.g., 0 to 255). Also, the ultrasound apparatus 1000 may receive a selection of a partial color range (e.g., -20 to 20). The ultrasound apparatus 1000 may receive a selection of a partial speed range (e.g., 7 m/s or more).

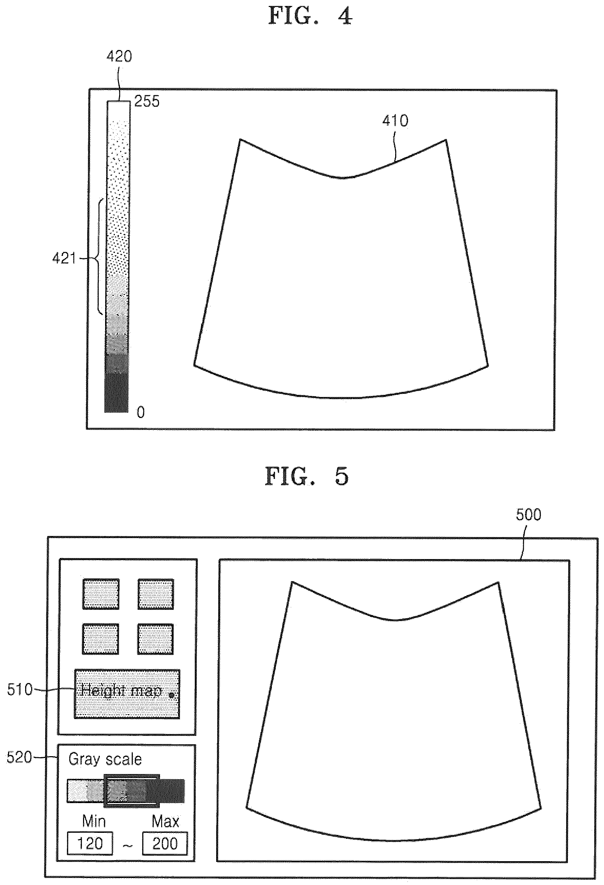

According to an exemplary embodiment, the ultrasound apparatus 1000 may receive a user selection of a predetermined range through an image pixel information map. For example, the ultrasound apparatus 1000 may display an image pixel information map, in which the image pixel information is gradually represented, on the screen. Then, the ultrasound apparatus 1000 may receive a user input for selecting the first range through the image pixel information map. This will be described later in detail with reference to FIG. 4.

According to an exemplary embodiment, the image pixel information map may include, but is not limited to, at least one of a gray scale map, a color scale map, an elasticity map, and a speed map. The gray scale map may be a map in which a brightness level is represented gradually from a white color to a black color. The color scale map may be a map in which a color change is gradually represented. The elasticity map may be a map in which elasticity is gradually represented, and the speed map may be a map in which a speed change is gradually represented.

According to an exemplary embodiment, the ultrasound apparatus 1000 may receive a selection of at least one range of the image pixel information through a graphical user interface (GUI). For example, the ultrasound apparatus 1000 may display a setting window for setting a partial range of the image pixel information on the screen. Then, the ultrasound apparatus 1000 may receive a selection of the first range of the image pixel information through the setting window. This will be described later in detail with reference to FIG. 5.

In operation S230, the ultrasound apparatus 1000 may extract partial ultrasound image data corresponding to at least one range of the image pixel information. According to an exemplary embodiment, the ultrasound apparatus 1000 may extract first ultrasound image data corresponding to the first range of the image pixel information from among the ultrasound image data. For example, the ultrasound apparatus 1000 may extract first ultrasound image data corresponding to a predetermined brightness range (e.g., 100 to 150). Also, the ultrasound apparatus 1000 may extract first ultrasound image data corresponding to a predetermined color range (e.g., -20 to 20). The ultrasound apparatus 1000 may extract first ultrasound image data corresponding to a partial speed range (e.g., 7 m/s or more).

In operation S240, the ultrasound apparatus 1000 may three-dimensionally display an ultrasound image corresponding to the at least one range by using the partial ultrasound image data. For example, the ultrasound apparatus 1000 may three-dimensionally display a first ultrasound image corresponding to the first ultrasound image data by using the first ultrasound image data.

According to an exemplary embodiment, the ultrasound apparatus 1000 may convert first image pixel information acquired from the first ultrasound image data into height values by using a mapping table. For example, the ultrasound apparatus 1000 may convert predetermined brightness values (e.g., 100 to 150) acquired from the first ultrasound image data into height values. In this case, the height values may increase as the brightness values increase; however, embodiments are not limited thereto.

According to an exemplary embodiment, the ultrasound apparatus 1000 may determine a sign (e.g., a plus sign or a minus sign) of the height values based on movement direction information of a tissue or a bloodstream when the ultrasound image data is color Doppler image data or spectral Doppler image data. For example, when the movement direction of a tissue or a bloodstream is a first direction, the height value may be a positive value; and when the movement direction of a tissue or a bloodstream is a second direction, the height value may be a negative value.

Also, the ultrasound apparatus 1000 may determine a sign (e.g., a plus sign or a minus sign) of the height values with respect to other directional image data in addition to Doppler image data (e.g., color Doppler image data or spectral Doppler image data).

According to an exemplary embodiment, the ultrasound apparatus 1000 may two-dimensionally display other images except the first ultrasound image among the ultrasound image displayed based on the ultrasound image data. For example, the ultrasound apparatus 1000 may display a region with a speed of 7 m/s or more in a spectral Doppler image or a color Doppler image in a 3D height map and may display a region with a speed of less than 7 m/s in the spectral Doppler image or the color Doppler image as a 2D image. In this specification, the height map may refer to a 3D image that is displayed along a depth axis, a scan line axis, and a height axis.

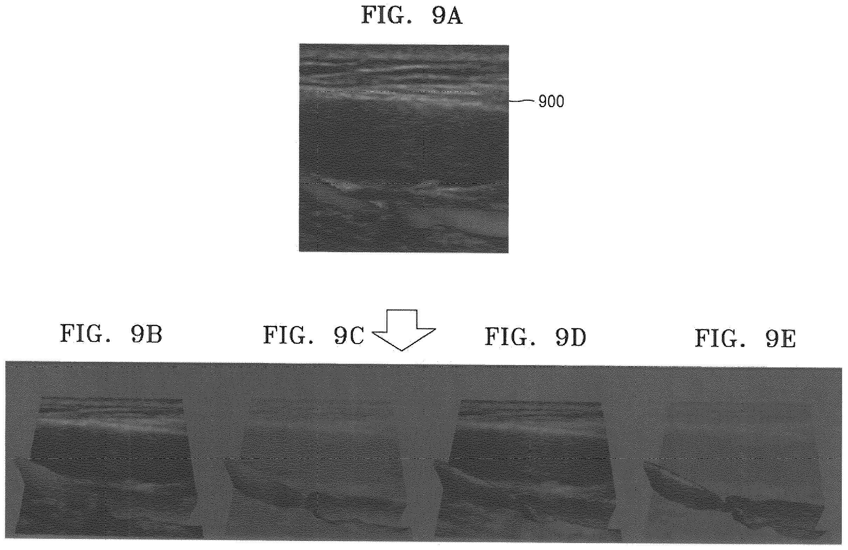

According to an exemplary embodiment, the ultrasound apparatus 1000 may transparently or semi-transparently display the other images except an ultrasound image corresponding to at least one range (e.g., the first range) among the entire ultrasound image displayed based on the ultrasound image data. This will be described later in detail with reference to FIG. 9.

According to an exemplary embodiment, the ultrasound apparatus 1000 may three-dimensionally display an ultrasound image corresponding to at least one range by using a light source-based rendering method. The light source-based rendering method (e.g., ray tracing) refers to a rendering method that traces a virtual visible ray to determine a color of a pixel. For example, the light source-based rendering method may process an object surface brightness, a light reflection effect, and a light refraction effect in the relationship between light and an object.

According to an exemplary embodiment, the ultrasound apparatus 1000 may receive a selection of at least one rendering method among a plurality of rendering methods and three-dimensionally display an ultrasound image corresponding to at least one range by using the selected rendering method. The rendering method described herein may include, but is not limited to, a shading-based rendering method, a light source-based rendering (e.g., ray tracing) method, a radiosity-based rendering method, a volume rendering method, an image-based rendering (IBR) method, and a non-photorealistic rendering (NPR) method. Hereinafter, for convenience of description, the shading-based rendering method and the light source-based rendering method (e.g., ray tracing) will be described as examples. The shading-based rendering method calculates the brightness of an object based on the properties of light.

According to an exemplary embodiment, when the user selects a plurality of ranges, the ultrasound apparatus 1000 may three-dimensionally display ultrasound images corresponding respectively to the plurality of ranges.

For example, the ultrasound apparatus 1000 may further receive a selection of a second range of the image pixel information. In this case, the ultrasound apparatus 1000 may further extract second ultrasound image data corresponding to the second range of the image pixel information from among the ultrasound image data. The ultrasound apparatus 1000 may display a second ultrasound image corresponding to the second ultrasound image data as a 3D image having height values, like the first ultrasound image.

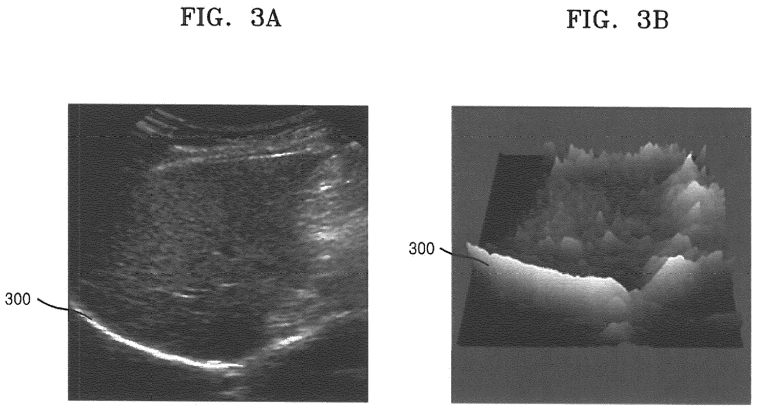

FIGS. 3A and 3B are diagrams illustrating a 3D ultrasound image having height values, according to an exemplary embodiment.

As illustrated in FIG. 3A, the ultrasound apparatus 1000 may acquire ultrasound image data of an object in a B mode and convert the ultrasound image data into height values. In this case, according to an exemplary embodiment, the ultrasound apparatus 1000 may set a height map mode based on a user input.

As illustrated in FIG. 3B, when the height map mode is set, the ultrasound apparatus 1000 may convert color values into height values. Then, the ultrasound apparatus 1000 may convert a 2D B mode image into a 3D height map by using the height values.

According to an exemplary embodiment, the ultrasound apparatus 1000 may provide an ultrasound image as a height map, so that the user may intuitively detect a brightness difference in the ultrasound image by using a height difference.

However, when the entire ultrasound image is provided as a height map, since a region such as a diaphragm 300 having high brightness values has a very high height values, images of other regions around the diaphragm 300 may be covered. In this case, the ultrasound apparatus 1000 may need to provide only a portion of the ultrasound image as a height map.

FIG. 4 is a diagram illustrating an operation of receiving a selection of at least one range through an image pixel information map, performed by the ultrasound apparatus 1000, according to an exemplary embodiment.

As illustrated in FIG. 4, the ultrasound apparatus 1000 may display an image pixel information map on one side of a 2D ultrasound image 410. For example, the ultrasound apparatus may provide a gray scale map 420.

The user may select a first brightness range 421 in order to detect height values through the gray scale map 420.

While FIG. 4 illustrates the gray scale map 420 as an example of the image pixel information map, embodiments are not limited thereto. Also, while FIG. 4 illustrates a bar-type image pixel information map, various types of image pixel information maps may be used.

FIG. 5 is a diagram illustrating a setting window for setting a partial range of image pixel information, according to an exemplary embodiment.

As illustrated in FIG. 5, the ultrasound apparatus 1000 may display a 2D ultrasound image 500 of an object. Then, the ultrasound apparatus 1000 may receive a user input for selecting a button 510 corresponding to a height map mode. The user input may vary according to embodiments. For example, the user input may include, but is not limited to, at least one of a key input, a touch input (e.g., a tap, a double tap, a touch & drag, a flick, or a swipe), a voice input, a motion input, and a multiple input.

When the user selects the button 510 corresponding to the height map mode, the ultrasound apparatus 1000 may provide a setting window 520 for setting a partial range of image pixel information. The ultrasound apparatus 1000 may receive a selection of a partial range of the image pixel information through the setting window from the user. For example, the user may select a partial brightness range (e.g., 120 to 200) among brightness values of 0 to 255.

Also, the ultrasound apparatus 1000 may directly receive a user input of a minimum value (e.g., 120) or a maximum value (e.g., 200) of a brightness range that is to be detected through a height map.

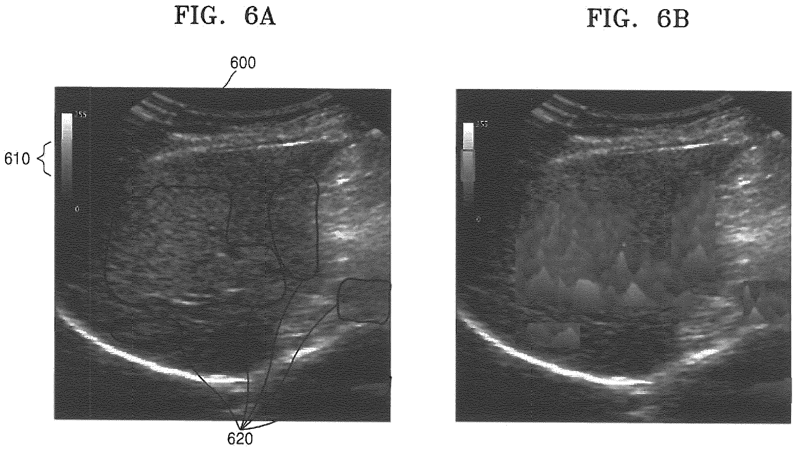

FIGS. 6A and 6B are diagrams illustrating a 3D B mode image obtained by converting ultrasound image data corresponding to a predetermined brightness range, according to an exemplary embodiment.

As illustrated in FIG. 6A, the ultrasound apparatus 1000 may display a 2D B mode image 600 and receive a selection of a brightness range 610. For example, the ultrasound apparatus 1000 may receive a selection of the brightness range 610 having brightness values of 120 to 180.

In this case, the ultrasound apparatus 1000 may extract ultrasound image data having the brightness values of 120 to 180. Then, the ultrasound apparatus 1000 may convert the brightness values of 120 to 180 into height values by using the extracted ultrasound image data.

As illustrated in FIG. 6B, the ultrasound apparatus 1000 may provide portions 620 having brightness values of 120 to 180 in the B mode image 600 as a 3D height map. The ultrasound apparatus 1000 may maintain a 2D form of other portions (e.g., a range with the brightness values of 0 to 119 and 181 to 255) in the ultrasound image.

According to an exemplary embodiment, the ultrasound apparatus 1000 provides a 3D height map for only a desired range of image pixel information, thereby increasing the user's detection of a desired image pixel information range (e.g., a desired brightness range, a desired speed range, a desired color range, and a desired elasticity range).

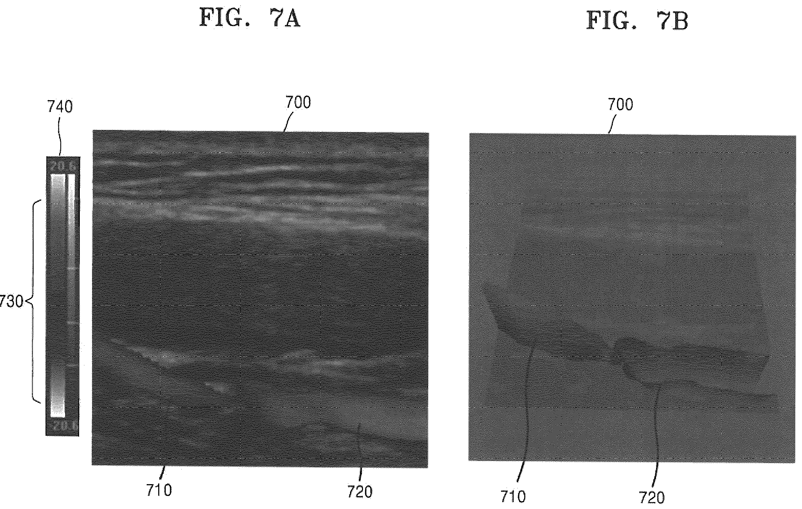

FIGS. 7A and 7B are diagrams illustrating a 3D color Doppler image obtained by converting ultrasound image data corresponding to a predetermined color range, according to an exemplary embodiment.

As illustrated in FIG. 7A, the ultrasound apparatus 1000 may provide a color Doppler image 700 of an object. In this case, the ultrasound apparatus 1000 may represent a bloodstream moving toward a probe with a red color 710 and represent a bloodstream moving away from the probe with a blue color 720. Also, the ultrasound apparatus 1000 may display a higher-speed bloodstream more brightly.

According to an exemplary embodiment, the ultrasound apparatus 1000 may receive a selection of a color range 730. The ultrasound apparatus 1000 may receive the selection of the color range 730 through a color scale map 740 or through a separate setting window. For example, the ultrasound apparatus 1000 may receive a selection of the color range 730 having brightness values of -18 to 16.

According to an exemplary embodiment, the ultrasound apparatus 1000 may extract ultrasound image data corresponding to the color range 730. Then, the ultrasound apparatus 1000 may convert predetermined color values (e.g., -18 to 16) into height values by using the extracted ultrasound image data.

As illustrated in FIG. 7B, the ultrasound apparatus 1000 may display portions 710 and 720 having color values of -18 to 16 in the color Doppler image 700 as a 3D height map. In this case, the ultrasound apparatus 1000 may display a red (710) region as a positive height value and display a blue (720) region as a negative height value. That is, the ultrasound apparatus 1000 displays the red (710) region as if it rises, and displays the blue (720) region as if it is recessed, thereby clearly displaying the movement direction of the bloodstream. Thus, according to an exemplary embodiment, even when the user has color weakness or color blindness, the user may accurately detect the movement direction of the bloodstream by using the height values.

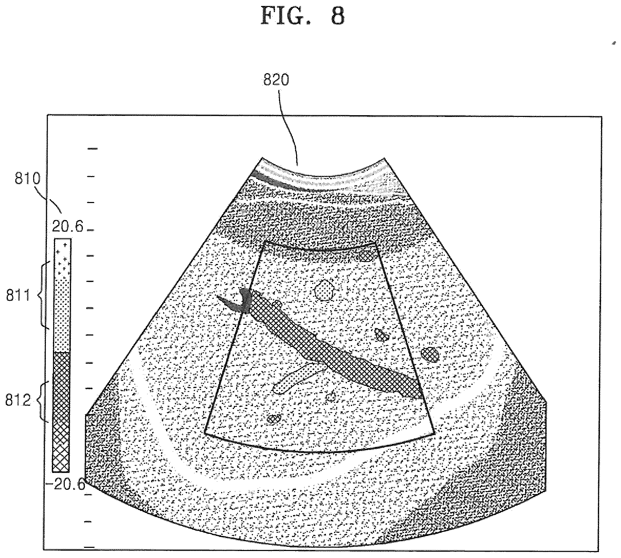

FIG. 8 is a diagram illustrating an operation of receiving a selection of a plurality of ranges of image pixel information, performed by the ultrasound apparatus 1000, according to an exemplary embodiment.

As illustrated in FIG. 8, the ultrasound apparatus 1000 may receive a selection of a plurality of color ranges 811 and 812 through a color scale map 810. For example, the ultrasound apparatus 1000 may receive a selection of a first color range (e.g., 4 to 16) 811 and a second color range (e.g., -4 to -11) 812.

According to an exemplary embodiment, the ultrasound apparatus 1000 may extract ultrasound image data corresponding respectively to the first color range 811 and the second color range 812. Then, the ultrasound apparatus 1000 may convert color values (e.g., 1 to 811) included in the first color range 811 and the second color range 812 into height values by using the extracted ultrasound image data.

By using the height values, the ultrasound apparatus 1000 may display a partial color region (e.g., 4 to 16 and -4 to -11) in a color Doppler image 820 as a 3D height map.

FIGS. 9A, 9B, 9C, 9D, and 9E are diagrams illustrating an operation of processing other images, except an ultrasound image corresponding to a selected range among the entire ultrasound image, to be transparent, performed by the ultrasound apparatus 1000, according to an exemplary embodiment.

As illustrated in FIG. 9A, according to an exemplary embodiment, the ultrasound apparatus 1000 may acquire a 2D ultrasound image 900 based on ultrasound image data. In this case, the ultrasound apparatus 1000 may three-dimensionally display an ultrasound image corresponding to at least one range (e.g., a first range) of image pixel information and may transparently or semi-transparently display the other images.

As illustrated in FIG. 9B, the ultrasound apparatus 1000 may display an ultrasound image corresponding to a predetermined color range as a 3D height map and may display the other images as opaque 2D images. In this case, the 3D height map may be covered by the opaque 2D images.

Thus, as illustrated in FIG. 9C, the ultrasound apparatus 1000 may display an ultrasound image corresponding to a predetermined color range as a 3D height map and may display the other images as transparent or semitransparent 2D images. In this case, the user may clearly detect the 3D height map since the 3D height map penetrates the transparent or semitransparent 2D images.

As illustrated in FIG. 9D, according to an exemplary embodiment, the ultrasound apparatus 1000 may display an ultrasound image corresponding to a predetermined color range as a 3D height map by using a light source-based rendering method and may display the other images as opaque 2D images.

As illustrated in FIG. 9E, according to an exemplary embodiment, the ultrasound apparatus 1000 may display an ultrasound image corresponding to a predetermined color range as a 3D height map by using a light source-based rendering method and may display the other images as transparent or semitransparent 2D images.

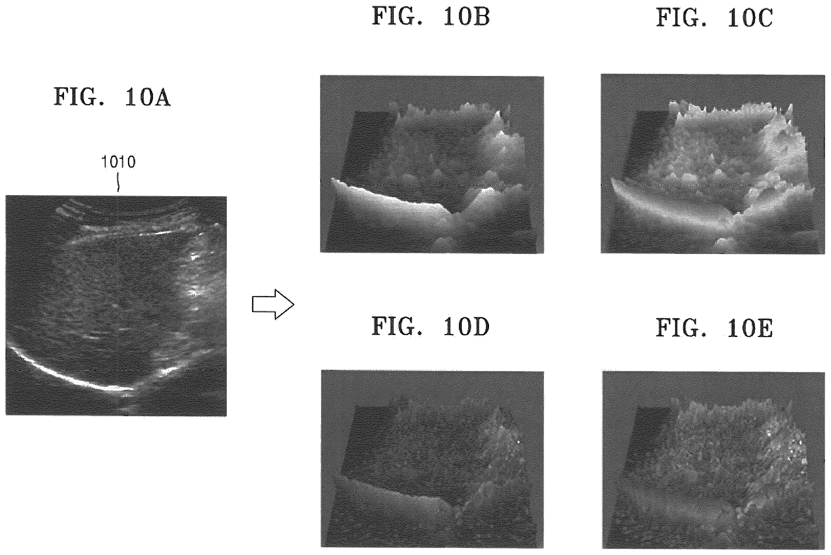

FIGS. 10A, 10B, 10C, 10D, and 10E are diagrams illustrating an operation of selecting a rendering method, performed by the ultrasound apparatus 1000, according to an exemplary embodiment.

As illustrated in FIG. 10A, according to an exemplary embodiment, the ultrasound apparatus 1000 may acquire a 2D ultrasound image 1010 of a hepatic portal vein. In this case, the ultrasound apparatus 1000 may receive a selection of at least one rendering method among a plurality of rendering methods. The ultrasound apparatus 1000 may three-dimensionally display an ultrasound image corresponding to at least one range (e.g., a first range) of image pixel information by the selected rendering method.

As illustrated in FIG. 10B, the ultrasound apparatus 1000 may receive a selection of a first rendering method that converts brightness values into height values based on a shading-based rendering method. In this case, the ultrasound apparatus 1000 may convert brightness values corresponding to at least one range (e.g., the first range) into height values by the first rendering method. For example, the ultrasound apparatus 1000 may display a 3D gray image of the hepatic portal vein by converting a bright portion into high height values and converting a dark portion into low height values.

As illustrated in FIG. 10C, the ultrasound apparatus 1000 may receive a selection of a second rendering method that converts brightness values into height values and color values based on a shading-based rendering method. In this case, the ultrasound apparatus 1000 may convert brightness values corresponding to at least one range (e.g., the first range) into height values and color values by the second rendering method. For example, the ultrasound apparatus 1000 may display a 3D color image of the hepatic portal vein by converting a bright portion into a red color having high height values and converting a dark portion into a blue color having low height values.

As illustrated in FIG. 10D, the ultrasound apparatus 1000 may receive a selection of a third rendering method that converts brightness values into height values based on a ray tracing-based rendering method. In this case, the ultrasound apparatus 1000 may display a 3D gray image of the hepatic portal vein by converting brightness values corresponding to at least one range (e.g., the first range) into height values by the third rendering method and calculating brightness values and transparency values at each sample point on a virtual ray.

As illustrated in FIG. 10E, the ultrasound apparatus 1000 may receive a selection of a fourth rendering method that converts brightness values into height values and color values based on a ray tracing-based rendering method. In this case, the ultrasound apparatus 1000 may display a 3D color image of the hepatic portal vein by converting brightness values corresponding to at least one range (e.g., the first range) into height values and color values by the fourth rendering method and calculating brightness values and transparency values at each sample point on a virtual ray.

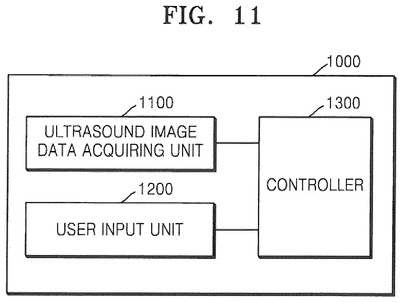

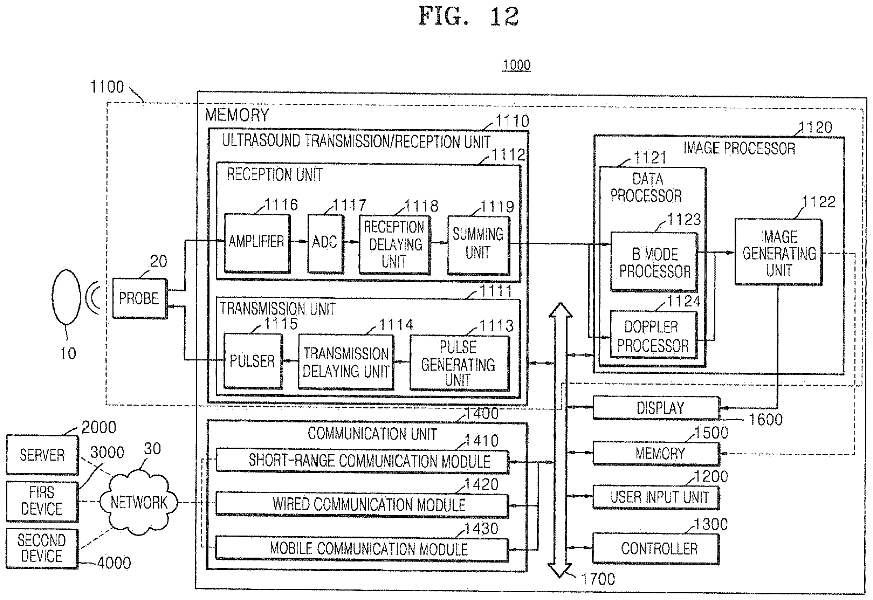

FIGS. 11 and 12 are block diagrams of the ultrasound apparatus 1000 according to an exemplary embodiment.

As illustrated in FIG. 11, the ultrasound apparatus 1000 according to an exemplary embodiment may include an ultrasound image data acquiring unit 1100, a user input unit 1200, and a controller 1300. However, all of the illustrated components are not necessary components. The ultrasound apparatus 1000 may include more or less components than the illustrated components. For example, as illustrated in FIG. 12, the ultrasound apparatus 1000 according to an exemplary embodiment may further include a communication unit 1400, a memory 1500, and a display 1600 in addition to the ultrasound image data acquiring unit 1100, the user input unit 1200, and the controller 1300. The above components may be connected through a bus 1700.

The above components will be described below.

The ultrasound image data acquiring unit 1100 according to an exemplary embodiment may acquire ultrasound image data of an object 10. The ultrasound image data according to an exemplary embodiment may be 2D ultrasound image data or 3D ultrasound image data of the object 10.

According to an exemplary embodiment, the ultrasound image data acquiring unit 1100 may include a probe 20, an ultrasound transmission/reception unit 1110, and an image processing unit 1120.

The probe 20 transmits an ultrasound signal to the object 10 according to a driving signal applied from the ultrasound transmission/reception unit 1110 and receives an echo signal reflected from the object 10. The probe 20 includes a plurality of transducers, and the plurality of transducers oscillate according to an electrical signal transmitted thereto and generate an ultrasound wave, that is, acoustic energy. Also, the probe 20 may be connected to a main body of the ultrasound apparatus 1000 by wire or wirelessly. According to embodiments, the ultrasound apparatus 1000 may include a plurality of probes 20. According to an exemplary embodiment, the probe 20 may include at least one of a one-dimensional (1 D) probe, a 1.5D probe, a 2D (matrix) probe, and a 3D probe.

A transmission unit 1111 supplies a driving signal to the probe 20 and includes a pulse generating unit 1113, a transmission delaying unit 1114, and a pulser 1115. The pulse generating unit 1113 generates pulses for forming transmission ultrasound waves according to a predetermined pulse repetition frequency (PRF), and the transmission delaying unit 1114 applies a delay time for determining transmission directionality to the pulses. The pulses to which a delay time is applied correspond to a plurality of piezoelectric vibrators included in the probe 20, respectively. The pulser 1115 applies a driving signal (or a driving pulse) to the probe 20 at a timing corresponding to each pulse to which a delay time is applied.

A reception unit 1112 generates ultrasound data by processing echo signals received from the probe 20 and may include an amplifier 1116, an analog-digital converter (ADC) 1117, a reception delaying unit 1118, and a summing unit 1119. The amplifier 1116 amplifies echo signals in each channel, and the ADC 1117 analog-digital converts the amplified echo signals. The reception delaying unit 1118 applies delay times for determining reception directionality to the digital-converted echo signals, and the summing unit 1119 generates ultrasound image data by summing the echo signals processed by the reception delaying unit 1118.

The image processing unit 1120 generates an ultrasound image by scan-converting ultrasound image data generated by the ultrasound transmission/reception unit 1110. The ultrasound image may include not only a gray-scale ultrasound image obtained by scanning the object according to an amplitude (A) mode, a brightness (B) mode, and a motion (M) mode, but also a Doppler image representing a motion of a moving object by using a Doppler effect. The Doppler image may include a bloodstream Doppler image (also referred to as a color Doppler image) representing a flow of blood, a tissue Doppler image representing a motion of a tissue, and a spectral Doppler image representing a movement speed of an object, in a waveform.

A B mode processing unit 1123 extracts B mode components from ultrasound image data and processes the B mode components. An image generating unit 1122 may generate an ultrasound image representing signal intensities as brightness based on the B mode components extracted by the B mode processing unit 1123.

Likewise, a Doppler processing unit 1124 may extract Doppler components from ultrasound image data, and the image generating unit 1122 may generate a Doppler image representing a motion of an object as colors or waveforms based on the extracted Doppler components.

The image generating unit 1122 according to an exemplary embodiment may generate a 3D ultrasound image through volume-rendering of volume data and may also generate an elasticity image that visualizes deformation of the object 10 due to a pressure.

In addition, the image generating unit 1122 may display various additional information in an ultrasound image by texts or graphics. For example, the image generating unit 1122 may add at least one annotation related to all or a portion of the ultrasound image to the ultrasound image. That is, the image generating unit 1122 may analyze the ultrasound image and add at least one annotation related to all or a portion of the ultrasound image to the ultrasound image based on the analysis result. Also, the image generating unit 1122 may add at least one annotation selected by the user to the ultrasound image.

The image processing unit 1120 may extract a region of interest (ROI) from the ultrasound image by using an image processing algorithm. In this case, the image processing unit 1120 may add a color, a pattern, or a frame to the ROI.

The image processing unit 1120 may convert image pixel information (e.g., brightness values, color values, speed values, elasticity values, amplitude values of a sound reflection signal, and sound impedance values) acquired from the ultrasound image data into height values. In this case, the image processing unit 1120 may convert a partial image pixel information range into height values.

The user input unit 1200 refers to a unit through which the user (e.g., sonographer) inputs data for controlling the ultrasound apparatus 1000. For example, the user input unit 1200 may include, but is not limited to, a keypad, a dome switch, a touch pad (e.g., a capacitive overlay type, a resistive overlay type, an infrared beam type, a surface acoustic wave type, an integral strain gauge type, or a piezoelectric type), a track ball, and a jog switch. For example, the user input unit 1200 may further include various input units such as an electrocardiogram measuring module, a breath measuring module, a voice recognition sensor, a gesture recognition sensor, a fingerprint recognition sensor, an iris recognition sensor, a depth sensor, and a distance sensor.

According to an exemplary embodiment, the user input unit 1200 may detect not only a real touch but also a proximity touch. The user input unit 1200 may detect a touch input (e.g., a touch & hold, a tap, a double tap, or a flick) to the ultrasound image. Also, the user input unit 1200 may detect a drag input from a point at which a touch input is detected. The user input unit 1200 may detect a multi-touch input (e.g., a pinch) to at least two points included in the ultrasound image.

According to an exemplary embodiment, the user input unit 1200 may receive a selection of a first range of the image pixel information acquired from the ultrasound image data. For example, the user input unit 1200 may receive a user input for selecting the first range through an image pixel information map (e.g., a gray scale map, a color scale map, an elasticity map, and a speed map) Also, the user input unit 1200 may receive a selection of the first range through a separate setting window.

The user input unit 1200 may receive a selection of a plurality of ranges of the image pixel information. For example, the user input unit 1200 may receive a selection of a first range and a second range of the image pixel information acquired from the ultrasound image data.

The user input unit 1200 may receive a selection of at least one rendering method from among a plurality of rendering methods.

The controller 1300 may control overall operations of the ultrasound apparatus 1000. For example, the controller 1300 may control overall operations of the ultrasound image data acquiring unit 1100, the user input unit 1200, the communication unit 1400, the memory 1500, and the display 1600.

According to an exemplary embodiment, the user input unit 1300 may extract first ultrasound image data corresponding to the first range of the image pixel information from among the ultrasound image data. The ultrasound image data may include at least one of brightness (B) mode image data, spectral Doppler image data, color Doppler image data, elasticity image data, and motion (M) mode image data. The controller 1300 may control the display 1600 to three-dimensionally display a first ultrasound image corresponding to the first ultrasound image data by using the first ultrasound image data.

The controller 1300 may control the display 1600 to display an image pixel information map in which the image pixel information is gradually represented. The image pixel information map may include at least one of a gray scale map, a color scale map, an elasticity map, and a speed map. Also, the controller 1300 may control the display 1600 to display a setting window for setting the first range of the image pixel information.

The controller 1300 may convert first image pixel information acquired from the first ultrasound image data into height values. The controller 1300 may determine a sign of the height values based on movement direction information of a tissue or a bloodstream when the ultrasound image data is color Doppler image data or spectral Doppler image data.

The controller 1300 may control the display 1600 to two-dimensionally display other images except the first ultrasound image among the ultrasound image displayed based on the ultrasound image data.

When a selection of the second range of the image pixel information is also received, the controller 1300 may also extract second ultrasound image data corresponding to the second range from among the ultrasound image data. The controller 1300 may control the display 1600 to three-dimensionally display a second ultrasound image corresponding to the second ultrasound image data by using the second ultrasound image data.

The controller 1300 may control the display 1600 to transparently or semi-transparently display the other images except the ultrasound image corresponding to at least one range among the entire ultrasound image displayed based on the ultrasound image data.

The controller 1300 may control the display 1600 to three-dimensionally display the ultrasound image corresponding to at least one range by using a light source-base rendering method.

The controller 1300 may control the display 1600 to three-dimensionally display the ultrasound image corresponding to at least one range by a rendering method selected by the user.

The communication unit 1400 may include one or more components for enabling communication between the ultrasound apparatus 1000 and a server 2000, between the ultrasound apparatus 1000 and a first device 3000, and between the ultrasound apparatus 1000 and a second device 4000. For example, the communication unit 1400 may include a short-range communication module 1410, a wired communication module 1420, and a mobile communication module 1430.

The short-range communication module 1410 refers to a module for short-range communication within a predetermined distance. Short-range communication technologies may include Wireless Local Area Network (LAN), Wi-Fi, Bluetooth, Bluetooth Low Energy (BLE), Ultra Wideband (UWB), Zigbee, Near Field Communication (NFC), Wi-Fi Direct (WFD), and Infrared Data Association (IrDA).

The wired communication module 1420 refers to a module for communication using electrical signals or optical signals. Examples of wired communication techniques according to an exemplary embodiment may include a pair cable, a coaxial cable, an optical fiber cable, and an Ethernet cable.

The mobile communication module 1430 communicates wireless signals with at least one of a base station, external devices (e.g., the first and second devices 3000 and 4000), and the server 2000 on a mobile communication network. Herein, the wireless signals may include voice call signals, video call signals, or various types of data for transmission and reception of text/multimedia messages.

The communication unit 1400 is connected by wire or wirelessly to a network 30 to communicate with an external device (e.g., the first device 3000 or the second device 4000) or the server 2000. The communication unit 1400 may exchange data with a hospital server or other medical apparatuses in a hospital connected through a Picture Archiving and Communication System (PACS). Also, the communication unit 1400 may perform data communication according to the Digital Imaging and Communications in Medicine (DICOM) standard.

The communication unit 1400 may transmit and receive data related to a diagnosis of the object 10, such as an ultrasound image, ultrasound image data, and Doppler image data of the object 10, through the network 30 and may also transmit and receive medical images captured by other medical devices, such as a CT image, a MRI image, and an X-ray image. In addition, the communication unit 1400 may receive information related to a diagnosis history or a treatment schedule of a patient from the server 2000 and utilize the information to diagnose the object 10.

The memory 1500 may store a program for processing of the controller 1300 and may store input/output data (e.g., ultrasound image data, a mapping table of brightness values and height values, a mapping table of speed values and height values, a mapping table of color values and height values, a mapping table of elasticity values and height values, information about a selected image pixel information range, ultrasound images, testee information, probe information, and body markers).

The memory 1500 may include at least one type of storage medium from among flash memory type, hard disk type, multimedia card micro type, card type memory (e.g., SD and XD memories), random access memory (RAM), static random access memory (SRAM), read-only memory (ROM), electronically erasable programmable read-only memory (EEPROM), programmable read-only memory (PROM), magnetic memory, magnetic disk, and optical disk. Also, the ultrasound apparatus 1000 may utilize a web storage or a cloud server that functions as the memory 1500 on the Internet.

The display 1600 may display information processed in the ultrasound apparatus 1000. For example, the display 1600 may display an ultrasound image or may display a user interface (UI) or a graphical user interface (GUI) related to a control panel.

The display 1600 may display a partial region of the ultrasound image three-dimensionally and display other regions two-dimensionally. For example, the display 1600 may display an ultrasound image corresponding to a partial range of the image pixel information as a 3D height map.

The display 1600 may display an image pixel information map in which the image pixel information is gradually represented. Also, the display 1600 may display a setting window for setting the first range of the image pixel information.

When a plurality of ranges of the image pixel information are selected, the display 1600 may display an ultrasound image corresponding to the plurality of ranges as a 3D height map.

When the display 1600 includes a touchscreen with a layer structure of a touch pad, the display 1600 may be used as an input device in addition to an output device. The display 1600 may include at least one of a liquid crystal display (LCD), a thin film transistor liquid crystal display (TFT-LCD), an organic light-emitting diode (OLED) display, a flexible display, a 3D display, and an electrophoretic display. Also, the ultrasound apparatus 1000 may include two or more displays 1600 according to embodiments.

The methods according to the exemplary embodiments may be embodied in the form of program commands executable through various computer means, which may be recorded on a computer-readable recording medium. The computer-readable recording medium may include program commands, data files, and data structures either alone or in combination. The program commands recorded on the computer-readable recording medium may be those that are especially designed and configured for the inventive concept, or may be those that are known and available to computer programmers skilled in the art. Examples of the computer-readable recording medium include magnetic recording media such as hard disks, floppy disks and magnetic tapes, optical recording media such as CD-ROMs and DVDs, magneto-optical recording media such as floptical disks, and hardware devices such as ROMs, RAMs and flash memories that are especially configured to store and execute program commands. Examples of the program commands include machine language codes that may be generated by a compiler, and high-level language codes that may be executed by a computer by using an interpreter.

As described above, according to the one or more of the above exemplary embodiments, the ultrasound apparatus 1000 provides a 3D height map about clinically meaningful measurement values, thereby improving convenience of an ultrasound diagnosis of the user. Also, an operation speed may be increased as compared with the case of providing an overall height map.

It should be understood that the exemplary embodiments described herein should be considered in a descriptive sense only and not for purposes of limitation. Descriptions of features or aspects within each exemplary embodiment should typically be considered as available for other similar features or aspects in other exemplary embodiments.

While one or more exemplary embodiments have been described with reference to the figures, it will be understood by those of ordinary skill in the art that various changes in form and details may be made therein without departing from the scope of the invention as defined by the following claims.

## Claims

1. An ultrasound image display method comprising:
acquiring ultrasound image data of an object;
receiving, via a graphical user interface (GUI), a selection of at least one range of image pixel information acquired from the ultrasound image data, wherein the GUI is displayed in a different area from an ultrasound image corresponding to the ultrasound image data;
extracting partial ultrasound image data corresponding to the at least one range from among the ultrasound image data; and
three-dimensionally displaying an ultrasound image corresponding to the at least one range based on the partial ultrasound image data,
wherein the at least one range comprises at least one from among a partial brightness range, a partial color range, and a partial speed range,
wherein the three-dimensional displaying of the ultrasound image comprises:
receiving a selection of at least one rendering method among a plurality of rendering methods; and
three-dimensionally displaying the ultrasound image corresponding to the at least one range by using the selected rendering method.

2. The ultrasound image display method of claim 1, wherein the ultrasound image data comprises at least one of brightness (B) mode image data, spectral Doppler image data, color Doppler image data, elasticity image data, and motion (M) mode image data.

3. The ultrasound image display method of claim 1, wherein the image pixel information acquired from the ultrasound image data comprises at least one of brightness information, speed information, color information, elasticity information, amplitude information of a sound reflection signal, and sound impedance information.

4. The ultrasound image display method of claim 1, wherein the receiving of the selection of the at least one range comprises:
displaying an image pixel information map, in which the image pixel information is gradually represented, on a screen; and
receiving a user input for selecting the at least one range through the image pixel information map.

5. The ultrasound image display method of claim 4, wherein the image pixel information map comprises at least one of a gray scale map, a color scale map, an elasticity map, and a speed map.

6. The ultrasound image display method of claim 1, wherein the receiving of the selection of the at least one range comprises:
displaying a setting window for setting the at least one range on a screen; and
receiving the selection of the at least one range through the setting window.

7. The ultrasound image display method of claim 1, wherein the three-dimensional displaying of the ultrasound image corresponding to the at least one range comprises converting image pixel information acquired from the partial ultrasound image data into height values.

8. The ultrasound image display method of claim 7, wherein the converting of the image pixel information into the height values comprises determining a sign of the height values based on movement direction information of a tissue or a bloodstream when the partial ultrasound image data is color Doppler image data or spectral Doppler image data.

9. The ultrasound image display method of claim 1, wherein the three-dimensional displaying of the ultrasound image corresponding to the at least one range comprises two-dimensionally displaying other images except the ultrasound image corresponding to the at least one range among the entire ultrasound image displayed based on the ultrasound image data.

10. The ultrasound image display method of claim 1, wherein the three-dimensional displaying of the ultrasound image corresponding to the at least one range comprises transparently or semi-transparently displaying other images except the ultrasound image corresponding to the at least one range among the entire ultrasound image displayed based on the ultrasound image data.

11. The ultrasound image display method of claim 1, wherein the three-dimensional displaying of the ultrasound image corresponding to the at least one range comprises three-dimensionally displaying the ultrasound image corresponding to the at least one range by using a light source-based rendering method.

12. The ultrasound image display method of claim 1, wherein the receiving of the selection of the at least one range comprises receiving a selection of a first range and a second range of the image pixel information acquired from the ultrasound image data.

13. An ultrasound apparatus (1000) comprising:
an ultrasound image data acquiring unit (1100) configured to acquire ultrasound image data of an object;
a user input unit (1200) configured to receive, via a graphical user interface (GUI), a selection of at least one range of image pixel information acquired from the ultrasound image data, wherein the GUI is displayed in a different area from an ultrasound image corresponding to the ultrasound image data; and
a controller (1300) configured to extract partial ultrasound image data corresponding to the at least one range from among the ultrasound image data, and control a display to three-dimensionally displaying an ultrasound image corresponding to the at least one range based on the partial ultrasound image data,
wherein the at least one range comprises at least one from among a partial brightness range, a partial color range, and a partial speed range, and
wherein the controller is further configured to:
receive a selection of at least one rendering method among a plurality of rendering methods, and
three-dimensionally display the ultrasound image corresponding to the at least one range by using the selected rendering method.

14. A non-transitory computer-readable recording medium that stores a program that, when executed by the ultrasound apparatus of claim 13, performs the ultrasound image display method of claim 1.

## Patentansprüche

1. Verfahren zum Anzeigen eines Ultraschallbilds, welches Folgendes aufweist:
Erlangen von Ultraschallbilddaten eines Objekts;
Empfangen einer Auswahl von wenigstens einem Bereich von Bildpixelinformationen, die von den Ultraschallbilddaten erlangt wurden über eine grafische Benutzerschnittstelle (GUI), wobei die GUI in einem unterschiedlichen Bereich wie ein Ultraschallbild entsprechend den Ultraschallbilddaten angezeigt wird;
Extrahieren teilweiser Ultraschallbilddaten entsprechend dem wenigstens einen Bereich unter den Ultraschallbilddaten; und
dreidimensionales Anzeigen eines Ultraschallbilds entsprechend dem wenigstens einen Bereich basierend auf den teilweisen Ultraschallbilddaten,
wobei der wenigstens eine Bereich wenigstens einen von einem teilweisen Helligkeitsbereich, einem teilweisen Farbbereich und einem teilweisen Geschwindigkeitsbereich aufweist,
wobei das dreidimensionale Anzeigen des Ultraschallbilds Folgendes aufweist:
Empfangen einer Auswahl von wenigstens einem Renderverfahren unter einer Vielzahl von Renderverfahren; und
dreidimensionales Anzeigen des Ultraschallbilds entsprechend dem wenigstens einen Bereich unter Verwendung des ausgewählten Renderverfahrens.

2. Verfahren zum Anzeigen eines Ultraschallbilds nach Anspruch 1, wobei die Ultraschalldaten wenigstens eine von Helligkeits- (B)-Modusbilddaten, Spektral-Dopplerbilddaten, Farb-Dopplerbilddaten, Elastizitätsbilddaten und Bewegungs-(M)-Modusbilddaten aufweisen.

3. Verfahren zum Anzeigen eines Ultraschallbilds nach Anspruch 1, wobei die Bildpixelinformationen, die von dem Ultraschallbild erlangt wurden, wenigstens eines von Helligkeitsinformationen, Geschwindigkeitsinformationen, Farbinformationen, Elastizitätsinformationen, Amplitudeninformationen eines Schallreflexionssignals und Schallimpedanzinformationen aufweisen.

4. Verfahren zum Anzeigen eines Ultraschallbilds nach Anspruch 1, wobei das Empfangen der Auswahl des wenigstens einen Bereichs Folgendes aufweist:
Anzeigen einer Bildpixelinformationskarte, in der die Bildpixelinformationen schrittweise dargestellt sind, auf einem Bildschirm; und
Empfangen einer Benutzereingabe zum Auswählen des wenigstens einen Bereichs durch die Bildpixelinformationskarte.

5. Verfahren zum Anzeigen eines Ultraschallbilds nach Anspruch 4, wobei die Bildpixelinformationskarte wenigstens eines einer Graustufenkarte, einer Farbstufenkarte, einer Elastizitätskarte und einer Geschwindigkeitskarte aufweist.

6. Verfahren zum Anzeigen eines Ultraschallbilds nach Anspruch 1, wobei das Empfangen der Auswahl des wenigstens einen Bereichs Folgendes aufweist:
Anzeigen eines Festlegefensters zum Festlegen des wenigstens einen Bereichs auf einem Bildschirm; und
Empfangen der Auswahl des wenigstens einen Bereichs durch das Festlegefenster.

7. Verfahren zum Anzeigen eines Ultraschallbilds nach Anspruch 1, wobei das dreidimensionale Anzeigen des Ultraschallbilds entsprechend dem wenigstens einen Bereich das Konvertieren von Pixelinformationen, die von den teilweisen Ultraschallbilddaten erlangt wurden, in Höhenwerte aufweist.

8. Verfahren zum Anzeigen eines Ultraschallbilds nach Anspruch 7, wobei das Konvertieren der Bildpixelinformationen in die Höhenwerte das Ermitteln eines Vorzeichens der Höhenwerte basierend auf Bewegungsrichtungsinformationen eines Gewebes oder eines Blutstroms aufweist, wenn die teilweisen Ultraschallbilddaten Farb-Dopplerbilddaten oder Spektral-Dopplerbilddaten sind.

9. Verfahren zum Anzeigen eines Ultraschallbilds nach Anspruch 1, wobei das dreidimensionale Anzeigen des Ultraschallbilds entsprechend dem wenigstens einen Bereich das zweidimensionale Anzeigen anderer Bilder mit Ausnahme des Ultraschallbilds entsprechend dem wenigstens einen Bereich unter dem gesamten Ultraschallbild aufweist, das basierend auf den Ultraschallbilddaten angezeigt wird.

10. Verfahren zum Anzeigen eines Ultraschallbilds nach Anspruch 1, wobei das dreidimensionale Anzeigen des Ultraschallbilds entsprechend dem wenigstens einen Bereich das transparente oder halbtransparente Anzeigen anderer Bilder mit Ausnahme des Ultraschallbilds entsprechend dem wenigstens einen Bereich unter dem gesamten Ultraschallbild aufweist, das basierend auf den Ultraschallbilddaten angezeigt wird.

11. Verfahren zum Anzeigen eines Ultraschallbilds nach Anspruch 1, wobei das dreidimensionale Anzeigen des Ultraschallbilds entsprechend dem wenigstens einen Bereich das dreidimensionale Anzeigen des Ultraschallbilds entsprechend dem wenigstens einen Bereich durch Verwendung eines auf einer Lichtquelle basierenden Renderverfahrens aufweist.

12. Verfahren zum Anzeigen eines Ultraschallbilds nach Anspruch 1, wobei das Empfangen der Auswahl des wenigstens einen Bereichs das Empfangen einer Auswahl eines ersten Bereichs und eines zweiten Bereichs der Bildpixelinformationen aufweist, die von den Ultraschallbilddaten erlangt wurden.

13. Ultraschallvorrichtung (1000), welche Folgendes aufweist:
eine Ultraschalldatenerlangungseinheit (1100), die dafür vorgesehen ist, Ultraschallbilddaten eines Objekts zu erlangen;
eine Benutzereingabeeinheit (1200), die dafür vorgesehen ist, über eine grafische Benutzerschnittstelle (GUI) eine Auswahl von wenigstens einem Bereich von Bildpixelinformationen, die von den Ultraschallbilddaten erlangt wurden, zu empfangen, wobei die GUI in einem unterschiedlichen Bereich eines Ultraschallbilds entsorechend den Ultraschallbilddaten angezeigt wird: und
ein Steuergerät (1300), das dafür vorgesehen ist, teilweise Ultraschallbilddaten entsprechend dem wenigstens einen Bereich unter der Vielzahl der Ultraschallbilddaten zu extrahieren und eine Anzeige zu steuern, um ein Ultraschallbild entsprechend dem wenigstens einen Bereich basierend auf den teilweisen Ultraschallbilddaten dreidimensional anzuzeigen,
wobei der wenigstens eine Bereich wenigstens einen von einem teilweisen Helligkeitsbereich, einem teilweisen Farbbereich und einem teilweisen Geschwindigkeitsbereich aufweist, und
wobei das Steuergerät des Weiteren für Folgendes vorgesehen ist:
Empfangen einer Auswahl von wenigstens einem Renderverfahren unter einer Vielzahl von Renderverfahren; und
dreidimensionales Anzeigen des Ultraschallbilds entsprechend dem wenigstens einen Bereich unter Verwendung des ausgewählten Renderverfahrens.

14. Nicht flüchtiges, computerlesbares Aufzeichnungsmedium, das ein Programm speichert, das, wenn es von der Ultraschallvorrichtung nach Anspruch 13 ausgeführt wird, das Verfahren zum Anzeigen des Ultraschallbilds nach Anspruch 1 durchführt.

## Revendications

1. Procédé d'affichage d'une image ultrasonore comprenant :
l'acquisition des données de l'image ultrasonore d'un objet ;
la réception, via une interface utilisateur graphique (GUI), d'une sélection d'au moins une plage d'informations des pixels de l'image acquises des données de l'image ultrasonore, dans laquelle la GUI est affichée dans une zone différente de celle d'une image ultrasonore correspondant aux données de l'image ultrasonore ;
l'extraction de données partielles de l'image ultrasonore correspondant à ladite au moins une plage parmi les données de l'image ultrasonore ; et
l'affichage tridimensionnel d'une image ultrasonore correspondant à ladite au moins une plage basée sur les données partielles de l'image ultrasonore,
dans laquelle ladite au moins une plage comprend au moins l'une d'une plage de luminosités partielle, d'une plage de couleurs partielle et d'une plage de vitesses partielle,
dans laquelle l'affichage tridimensionnel de l'image ultrasonore comprend :
la réception d'une sélection d'au moins une méthode de rendu parmi une pluralité de méthodes de rendu ; et
l'affichage tridimensionnel de l'image ultrasonore correspondant à ladite au moins une plage en utilisant la méthode de rendu sélectionnée.

2. Procédé d'affichage d'une image ultrasonore selon la revendication 1, dans laquelle les données de l'image ultrasonore comprennent au moins l'une des données de l'image en mode luminosité (B), des données de l'image Doppler spectrale, des données de l'image Doppler couleurs, des données de l'image d'élasticité, et des données de l'image en mode mouvement (M).

3. Procédé d'affichage d'une image ultrasonore selon la revendication 1, dans laquelle les informations des pixels de l'image acquises des données de l'image ultrasonore comprennent au moins l'une des informations de luminosité, des informations de vitesse, des informations de couleur, des informations d'élasticité, des informations d'amplitude d'un signal de réflexion du son, et des informations d'impédance du son.

4. Procédé d'affichage d'une image ultrasonore selon la revendication 1, dans laquelle la réception de la sélection de ladite au moins une plage comprend :
l'affichage d'une carte d'informations des pixels de l'image, dans laquelle les informations des pixels de l'image sont représentées graduellement, sur un écran, et
la réception d'une entrée utilisateur pour la sélection de ladite au moins une plage dans la carte d'informations des pixels de l'image.

5. Procédé d'affichage d'une image ultrasonore selon la revendication 4, dans laquelle la carte d'informations des pixels de l'image comprend au moins l'une d'une carte d'échelle de gris, d'une carte d'échelle de couleurs, d'une carte d'élasticités et d'une carte de vitesses.

6. Procédé d'affichage d'une image ultrasonore selon la revendication 1, dans laquelle la réception de la sélection de ladite au moins une plage comprend :
l'affichage d'une fenêtre de réglage pour le réglage de ladite au moins une plage sur un écran ; et
la réception de la sélection de ladite au moins une plage par la fenêtre de réglage.

7. Procédé d'affichage d'une image ultrasonore selon la revendication 1, dans laquelle l'affichage tridimensionnel de l'image ultrasonore correspondant à ladite au moins une plage comprend la conversion des informations des pixels de l'image acquises des données partielles de l'image ultrasonore en valeurs de hauteur.

8. Procédé d'affichage d'une image ultrasonore selon la revendication 7, dans laquelle la conversion des informations des pixels de l'image en valeurs de hauteur comprend la détermination d'un signe des valeurs de hauteur basé sur les informations de direction de mouvement d'un tissu ou d'une circulation sanguine lorsque les données partielles de l'image ultrasonore sont des données d'une image Doppler couleurs ou des données d'une image Doppler spectrale.

9. Procédé d'affichage d'une image ultrasonore selon la revendication 1, dans laquelle l'affichage tridimensionnel de l'image ultrasonore correspondant à ladite au moins une plage comprend l'affichage bidimensionnel d'autres images à l'exception de l'image ultrasonore correspondant à ladite au moins une plage parmi l'image ultrasonore entière affichée sur la base des données de l'image ultrasonore.

10. Procédé d'affichage d'une image ultrasonore selon la revendication 1, dans laquelle l'affichage tridimensionnel de l'image ultrasonore correspondant à ladite au moins une plage comprend l'affichage transparent ou semi-transparent d'autres images à l'exception de l'image ultrasonore correspondant à ladite au moins une plage parmi l'image ultrasonore entière affichée sur la base des données de l'image ultrasonore.

11. Procédé d'affichage d'une image ultrasonore selon la revendication 1, dans laquelle l'affichage tridimensionnel de l'image ultrasonore correspondant à ladite au moins une plage comprend l'affichage tridimensionnel de l'image ultrasonore correspondant à ladite au moins une plage en utilisant une méthode de rendu basée sur une source de lumière.

12. Procédé d'affichage d'une image ultrasonore selon la revendication 1, dans laquelle la réception de la sélection de ladite au moins une plage comprend la réception d'une sélection d'une première plage et d'une seconde plage d'informations des pixels de l'image acquises des données de l'image ultrasonore.

13. Appareil à ultrasons (1000) comprenant :
une unité d'acquisition des données de l'image ultrasonore (1100) configurée pour acquérir des données de l'image ultrasonore d'un objet ;
une unité d'entrée (1200) configurée pour recevoir, via une interface utilisateur graphique (GUI), une sélection d'au moins une plage d'informations des pixels de l'image acquises des données de l'image ultrasonore, dans laquelle la GUI est affichée dans une zone différente de celle d'une image ultrasonore correspondant aux données de l'image ultrasonore ; et
un contrôleur (1300) configuré pour extraire des données partielles de l'image ultrasonore correspondant à ladite au moins une plage parmi les données de l'image ultrasonore, et commander un affichage pour l'affichage tridimensionnel d'une image ultrasonore correspondant à ladite au moins une plage basée sur les données partielles de l'image ultrasonore,
dans laquelle ladite au moins une plage comprend au moins l'une d'une plage de luminosités partielle, d'une plage de couleurs partielle et d'une plage de vitesses partielle, et
dans laquelle le contrôleur est en outre configuré pour :
recevoir une sélection d'au moins une méthode de rendu parmi une pluralité de méthodes de rendu ; et
afficher de manière tridimensionnelle l'image ultrasonore correspondant à ladite au moins une plage en utilisant la méthode de rendu sélectionnée.

14. Support d'enregistrement non transitoire lisible par ordinateur qui enregistre un programme qui, lorsqu'il est exécuté par l'appareil à ultrasons de la revendication 13, applique le procédé d'affichage d'une image ultrasonore de la revendication 1.
